# EUROPEAN PATENT SPECIFICATION

(11) **EP 3 840 410 B1**
(45) Date of publication and mention of the grant of the patent: **24.01.2024**
(21) Application number: 19216899.5
(22) Date of filing: 17.12.2019
(51) Int. Cl.: A61B 5/024, H04R 25/00, A61B 5/00, H04R 1/10

(54) **HEART RATE MEASUREMENTS WITH HEARING DEVICE USING IN/AT/ON-EAR SENSOR, SECOND BODY SENSOR AND APP**
HERZFREQUENZMESSUNGEN MIT HÖRVORRICHTUNG MIT IN/AN/AUF DEM OHR SENSOR, ZWEITEM KÖRPERSENSOR UND RECHNERNANWENDUNG
MESURES DE FRÉQUENCE CARDIAQUE À L'AIDE D'UN APPAREIL AUDITIF SITUÉ DANS/À/SUR L'OREILLE, D' UN SECOND CAPTEUR CORPOREL ET D'UNE APPLICATION

(43) Date of publication of application: 23.06.2021
(73) Proprietor: GN Hearing A/S, 2750 Ballerup (DK)
(72) Inventor: GOLDMAN, Tomasz, DK-2750 Ballerup (DK)
(74) Representative: GN Store Nord A/S

(56) References cited:
- EP-A1- 2 887 692
- US-A1- 2017 027 521
- US-A1- 2017 071 489

## Description

### FIELD

The present disclosure relates to a hearing device, system, an application and a method, performed in an electronic device, for monitoring correct placement of a first physiologic sensor in/on/at an ear of a user. The first physiologic sensor is arranged in a hearing device. The first physiologic sensor in the hearing device is configured for monitoring the heart activity, such as heart rate, of the user wearing the hearing device.

### BACKGROUND

Hearing devices can be worn by a user for allowing the user to receive audio signals. Hearing devices may be hearing aids which compensates for a user's hearing loss. Hearing aids may be used by elderly people having a hearing impairment. Sometimes elderly people also suffer from other conditions, such as heart diseases. If a person is having a heart disease, it may be advantageous for that person to monitor his/her heart rate. Heart rate can be monitored using different devices known as heart rate monitors or sensors. A heart rate sensor can be implemented in a hearing device for monitoring the heart rate in/on/at the ear of the user.

If a hearing device comprises a heart rate sensor for measuring the heart rate e.g. in the ear of the user, it is important or even necessary that the heart rate sensor is placed correctly in the ear such that correct heart rate measurements in the ear can be performed.

However, it may be difficult for hearing device user's to place a hearing device comprising a heart rate sensor correctly in the ear for obtaining suitable heart rate monitoring.

In the prior art, it is known according to the patent application EP2887692A1, a headphone system comprising a physiological sensor and a method of fitting the headphone system. The measured physiological data is processed to output a fitting parameter which is evaluated to indicate whether the headphone is properly positioned and to notify the user of in dependence of the fitting parameter. It is also known according to the patent application US2017/0071489A1, a heart rate monitoring system having at least one primary heart rate sensor for measuring a heart rate and a secondary sensor. The system uses a model unit for estimating or predicting a heart rate of a user based on the data of the secondary sensor. The measured heart rate and the estimated or predicted heart rate are correlated with each other to differentiate the measured heart rate beats from artifacts.

Thus, there is a need for an improved system and method for assisting a user to correctly place a hearing device comprising a heart rate sensor in/on/at the user's ear.

### SUMMARY

According to the invention, it is provided a method according to claim 1.

It is an advantage of the method that it can be used for assisting a user to correctly place a hearing device comprising a sensor for measuring heart rate in/on/at the user's ear. If a hearing device comprises a heart rate sensor for measuring the heart rate in/on/at the ear of the user, it is important or even necessary that the heart rate sensor is placed correctly in/on/at the ear such that correct heart rate measurements in/on/at the ear can be performed. Since it can be difficult for hearing device user's to place a hearing device comprising a heart rate sensor correctly in/on/at his/her ear for obtaining suitable heart rate monitoring, it is an advantage of the method that it provides for monitoring correct placement of the heart rate sensor in/on/at the ear.

Preferable aspects of the method are set forth in the dependent claims.

The method is for monitoring correct placement of a first physiologic sensor in/on/at an ear of a user. It is an advantage of the method that the monitoring may comprise determining whether the first physiologic sensor is placed correctly in/on/at the ear of the user. The monitoring may comprise performing measurements of the heart rate of the user. The method may comprise determining the quality of the fit of the physiologic sensor in/on/at the ear of the user. It is an advantage that by means of the method, it is possible to advise the user to change the orientation/location of the physiologic sensor in/on/at the ear.

The first physiologic sensor of claim 1 is arranged in a hearing device. The first physiologic sensor in the hearing device is configured for monitoring the heart rate of the user wearing the hearing device. The heart rate may also be known as the pulse, and the heart rate may be monitored in the form of a photoplethysmographic signal. The first physiologic sensor may be a sensor which is configured to detect the blood volume changes, in the microvascular bed of tissue, being representative for the heart rate of a user, as the heart rate is equal or close to the pulse measured at any peripheral point. The first physiologic sensor may be a photoplethysmographic (PPG) sensor.

It is an advantage to implement a first physiologic sensor, which is configured for monitoring the heart rate of the user, with a hearing device, since a user who is already a hearing device user, e.g. because of a hearing loss which is compensated by the hearing device, will have the benefit of both hearing loss compensation and heart rate monitoring in one device, i.e. in the hearing device.

It is an advantage that by monitoring the placement of the first physiologic sensor in the ear of the user, it is possible to measure whether the first physiologic sensor is placed correctly in/on/at the ear of the user. The first physiologic sensor should be placed in a correct or optimal position in/on/at the ear where the first physiologic sensor can obtain the heart rate measurements. For example, the first physiologic sensor should be placed close to the skin of the ear, such as close to an ear wall, e.g. an ear canal, concha, concha skin, or concha wall of the ear, for being able to obtain suitable, or the best possible, heart rate measurements. Thus, it is an advantage that if it is detected that the first physiologic sensor is not placed correctly in/on/at the ear of the user, the user can be notified to change the position of the first physiologic sensor in the ear, such that an optimal or better placement of the first physiologic sensor can be achieved.

It is an advantage that when the first physiologic sensor data match/are similar to the second physiologic data, then the first physiologic sensor is assumed to measure correct and thereby able to correctly monitor the heart rate in the ear of the user.

It is an advantage that when it is detected that the first physiologic sensor is placed correctly in/on/at the ear of the user, then reference physiologic sensor data of the heart rate of the user is determined. These reference physiologic sensor data can be used to detect if the heart rate or pulse of the user becomes abnormal or changes more than a threshold, in which case the user can be notified to contact a medical doctor.

The method may comprise detecting or obtaining information that the hearing device is inserted in/on/at an ear of the user.

The method comprises receiving first physiologic sensor data from the first physiologic sensor. The first physiologic sensor data are indicative of a first physiologic sensor signal. The first physiologic sensor data may be received in the electronic device by means of a connection, wireless or wired, with the hearing device. Light emitted from a light emitter of the first physiologic sensor may be directed to the skin of the ear canal or ear wall, e.g. the skin of the concha or concha wall. Some of the light may be absorbed by the tissue and some may be scattered back to the light transceiver. As some of this received light is affected by the pulsating blood in the blood vessels, the heart rate can be determined.

The method comprises receiving second physiologic sensor data from a second physiologic sensor provided in an external device. The external device is configured for detecting the heart rate of the user using the second physiologic sensor. The second physiologic data are indicative of a second physiologic sensor signal. The second physiologic sensor data may be received in the electronic device by means of a connection, wireless or wired, with the external device.

The second physiologic sensor data may be obtained from a different physical point or part or location on the user's body or skin than where the first physiologic sensor data is obtained. The first physiologic sensor data is obtained in/on/at the ear of the user. The second physiologic sensor data may be obtained e.g. at the chest or wrist or finger of the user. The first physiologic sensor data and the second physiologic sensor data are configured to be detected simultaneously.

The external device may be the electronic device, e.g. a smartphone, tablet, pc, computer etc., in which case the electronic device is configured for obtaining the second physiologic sensor data of the user.

When the external device is the electronic device, the second physiologic sensor is provided in the electronic device. The electronic device may be a smartphone, and the second physiologic sensor may comprise a camera lens and an LED light source of the smartphone. The second physiologic sensor may be configured for detecting the pulse in the user's fingertip, when the user places his/her fingertip on the camera lens and LED light source.

The external device may be a chest strap or pulse watch etc. which is configured for obtaining physiologic sensor data of the user. The chest strap is configured to obtain physiologic sensor data from the chest or the skin of the chest of the user. The pulse watch is configured to obtain physiologic sensor data from the wrist or the skin of the wrist of the user. Chest straps and pulse watches are typically used by a user to measure his/her own heart rate when performing sports.

The external device may be a medical finger sensor which is configured for obtaining physiologic sensor data of the user. The medical finger sensor is configured to obtain physiologic sensor data from the finger or the skin of the finger of the user. A medical finger sensor is typically used to measure a patient's heart rate by medical doctors in hospitals.

The method comprises comparing the first physiologic sensor data from the first physiologic sensor with the second physiologic sensor data from the second physiologic sensor. Comparing the first physiologic sensor data with the second physiologic sensor data may be performed in a comparator or a processing unit of the electronic device.

The method comprises determining whether the first physiologic sensor data match the second physiologic sensor data. Match may be understood as matches within a tolerance of 2-3 beats-per-minute. There may be no exact match because the heart rate is averaged over 1-10 beats. Thus, it is determined if the measured pulse in the ear of the user matches the measured pulse from the other physiologic sensor on the user. The first physiologic sensor data may match the second physiologic sensor data, if the first physiologic sensor data and the second physiologic sensor data are the same or similar or corresponding or within a threshold value of each other.

The first physiologic sensor data may not match the second physiologic sensor data, if the first physiologic sensor data and the second physiologic sensor data are different or varies too much from each other, or varies more than a threshold value from each other or there are too much noise or too much variability in the sensor data, or if the first physiologic sensor data and the second physiologic sensor data are not the same or not similar or not corresponding or not within a threshold value of each other. According to the method, it is assumed that if the first physiologic sensor data does not match the second physiologic sensor data, it is because the first physiologic sensor is not placed correctly in/on/at the ear of the user.

According to the method, it is assumed that if the first physiologic sensor data does match the second physiologic sensor data, it is because the first physiologic sensor is placed correctly in/on/at the ear of the user.

For obtaining reliable first physiologic sensor data and second physiologic sensor data, the user should sit or lie still, i.e. the user should not be moving, when the first physiologic sensor data and the second physiologic sensor data are obtained, such as received, determined, retrieved and/or measured. Thus, if the first physiologic sensor data does not match the second physiologic sensor data it may be because the hearing device is wrongly placed, or the user is moving, e.g. not sitting still, or the user is eating or talking or chewing gum.

The method comprises in accordance with a determination that the first physiologic sensor data match the second physiologic sensor data, setting a reference physiologic sensor data.

The reference physiologic sensor data may be a resting heart rate of the user. If the reference physiologic sensor data should be a resting heart rate of the user, the first physiologic sensor data and second physiologic sensor data should be obtained when the user is sitting still or being in rest as opposed to moving.

The method comprises in accordance with a determination that the first physiologic sensor data match the second physiologic sensor data, setting a reference physiologic sensor data based on the first physiologic sensor data and/or the second physiologic sensor data. Thus, if the first physiologic sensor data and the second physiologic sensor data match, then a reference physiologic sensor data is set.

The reference physiologic sensor data is based on the first physiologic sensor data and/or the second physiologic sensor data. The first physiologic sensor data may be set as the reference physiologic sensor data. The second physiologic sensor data may be set as the reference physiologic sensor data. The first physiologic sensor data and the second physiologic sensor data may be set as the reference physiologic sensor data, e.g. if they are similar or equal. The reference physiologic sensor data may be a combination or a mix of the first physiologic sensor data and the second physiologic sensor data.

If using a medical finger sensor as the second physiologic sensor, then the second physiologic sensor data may be set as the reference physiologic sensor data, since the medical finger sensor may be considered to be a precise PPG device and thereby the second physiologic sensor is considered to perform a correct measurement of the heart rate of the user. The PPG sensor is considered accurate if the finger and the person is resting.

An electrocardiogram (EKG) may be used as the second physiologic sensor. An Electrocardiography is the process of producing an electrocardiogram (ECG or EKG), a recording, i.e. a graph of voltage versus time, of the electrical activity of the heart using electrodes placed on the skin of the user. If using an EKG as the second physiologic sensor, then the second physiologic sensor data may be set as the reference physiologic sensor data, since the EKG may be considered to be a precise heart rate device and thereby the second physiologic sensor is considered to perform a correct measurement of the heart rate of the user.

Thus, it may be assumed that the second physiologic sensor data is correct, and thereby that the heart rate measured by the second physiologic sensor is correct, i.e. the actual/ true heart rate of the user, and therefore when the first physiologic sensor data match/are similar to the second physiologic data, then the first physiologic sensor may be assumed to measure correct and thereby able to correctly monitor the heart rate in the ear of the user.

If using a chest strap, a pulse watch or a camera lens and LED light source of the smartphone as the second physiologic sensor, then the second physiologic sensor data may be considered to be less precise, since these types of second physiologic sensors may not be calibrated. Thus, if using a chest strap, a pulse watch or a camera lens and LED light source of the smartphone as the second physiologic sensor, the reference physiologic sensor data may be set to be a combination or a mix of the first physiologic sensor data and the second physiologic sensor data. Alternatively, the reference physiologic sensor data may be set to be the first physiologic sensor data or the second physiologic sensor data. When using a less precise second physiologic sensor, like e.g. a chest strap, pulse watch or a camera lens and LED light source of the smartphone, the second physiologic sensor data may not be assumed to be correct, and then the values of the measured heart rate from the first and second physiologic sensors may be within a threshold value of each other, in order for there to be a match.

The method is for monitoring correct placement of a first physiologic sensor in/on/at an ear of a user. The monitoring of correct placement may comprise comparing values of first and second physiologic sensor data to check or ensure that these values are close enough to each other.

The monitoring of correct placement of a first physiologic sensor in/on/at an ear of a user may comprise a calibration of the first physiologic sensor and/or of the second physiologic sensor. For performing a calibration, the method comprises using a reference device which is assumed to have a known deviation. The reference device may be the second physiologic sensor or another physiologic sensor.

The method comprises in accordance with a determination that the first physiologic sensor data do not match the second physiologic sensor data, providing a notification regarding adjusting the position of the first physiologic sensor in the ear of the user. If the first physiologic sensor data and the second physiologic sensor data do not match, it may be assumed that the first physiologic sensor is not correctly placed in/on/at the ear of the user. A notification may be provided to the user. The notification may comprise instructions to the user for adjusting the position of the first physiologic sensor in his/her ear. The first physiologic sensor may be implemented in the hearing device, thus the notification to the user may regard adjusting the position of the hearing device, whereby the first physiologic sensor in the hearing device is also adjusted in position relative to the ear of the user. The purpose of the notification is to make the user adjust the position of the first physiologic sensor, such that the first physiologic sensor is placed correctly to monitor the heart rate in/on/at the ear of the user.

The notification may be in the form of a recommendation, an error message, and/or instructions to adjust the position of the first physiologic sensor in/on/at the ear and/or adjust the position of the hearing device in/on/at the ear. The notification may comprise instructions to the user to change a fitting member of the hearing device to obtain a better fit of the hearing device in the ear. The notification may be directed to the user or to a hearing care professional (HCP) who is assisting the user with the hearing device. The notification may be instructions displayed on a first user interface of the electronic device, e.g. a graphical user interface on the user's smartphone, which is connected to the hearing device and optionally the external device. The notification may be visual, audio and/or tactile. The notification may comprise visual, audio and/or tactile notifications.

A visual notification may be a text alert stating e.g. "Please adjust position of your hearing device in the ear for improved monitoring of heart rate". A visual notification may be a text message stating e.g. "Please push your hearing device further into your ear canal", and/or "Please move your hearing device closer to an ear wall/the concha wall/the concha skin". A visual notification may comprise a picture of the desired orientation of the hearing device, e.g. showing that the end/side/face of the hearing device, where the first physiologic sensor is arranged, should point towards a specific ear wall, e.g. the ear canal, concha, concha skin, or concha wall, in/on the ear. The visual notification may be displayed in a graphical user interface of the electronic device.

An audio notification may be a sound alerting the user that the hearing device, and thereby the first physiologic sensor, is not correctly placed in/on/at the ear. The sound may be a tone or a voice speaking. The audio notification may be transmitted from a loudspeaker in the electronic device.

A tactile notification may be a haptic feedback or a vibration alerting the user that the hearing device, and thereby the first physiologic sensor, is not correctly placed in/on/at the ear. The tactile notification may be generated by a tactile output generator in the electronic device.

For obtaining reliable first physiologic sensor data and second physiologic sensor data, the user should sit or lie still, i.e. the user should not be moving, talking, eating, chewing etc. , when the first physiologic sensor data and the second physiologic sensor data are obtained, such as received, determined, retrieved and/or measured. Thus, if the first physiologic sensor data does not match the second physiologic sensor data it may be because the user is not sitting still but is moving. Thus, the notification may comprise instructions to the user to sit or lie still.

The method is performed in an electronic device. Thus, the electronic device is configured for performing the steps of the method. The electronic device may be a smartphone, a tablet, a pc, a computer or any other electronic device capable of performing a computer-implemented method, i.e. performing data processing, measurements, determination, calculations etc.

The electronic device and the hearing device are configured to be connected, such as electrically connected, such as wirelessly connected or connected by a wire. Thus, the electronic device and the hearing device may be in electronic communication with each other, such as for communicating data and/or electronic signals with other. The electronic device and the hearing device may be connected wirelessly by Bluetooth (BT), Bluetooth Low Energy (BLE), ZigBee, Wi-Fi, WLAN, LAN etc. The electronic device and the hearing device may be connected by wire, e.g. by means of USB connection or other hardware connection.

The hearing device may be a hearing aid for compensating for a hearing loss of the user. The hearing aid may be any hearing aid, such as a hearing aid of the in-the-ear type, such as in-the-canal type, such as completely-in-the-canal type of hearing aid, etc., a hearing aid of the behind-the-ear type, of the receiver-in-the-ear type of hearing aid, etc. The first physiologic sensor may be arranged in/on/at an in-the-ear unit of a hearing device.

The in-the-ear hearing aid typically comprises a shell, such as a polymer or plastic shell, in a shape configured to be provided in the ear, in the ear-canal or completely-in-the-canal of the ear of a user. The shell of an in-the-ear hearing aid may comprise a first end facing the eardrum of the user when the hearing aid is worn and a second end opposite the first end facing the surroundings when the hearing aid is worn. The shell may comprise a faceplate in the second end of the shell. The faceplate is a plate or cover closing the second end of the hearing aid shell. The hearing aid shell may be open in the second end and thus the faceplate provides a closing of the hearing aid shell. The faceplate may comprise one or components of the hearing aid. The faceplate may comprise a battery door. The faceplate may be detachable or removable from the hearing aid shell, e.g. for the purpose of changing the battery and/or replacing or repairing other components in the hearing aid shell.

The hearing device may comprise a microphone configured for converting an acoustic sound signal from a sound source into an audio signal. The audio signal is configured to be processed in a processing unit for compensation of the hearing loss of the user. The processed audio signal is configured to be converted into a processed acoustic signal by a receiver.

A further advantage of the method is that when assisting a user to correctly place a hearing device comprising a sensor for measuring heart rate in the user's ear, an optimal placement of the hearing aid in order to provide an optimal acoustic signal from the receiver in the hearing aid also may be obtained.

A yet further advantage is that the physiologic sensor can be used to determine whether the hearing device is inserted in the ear of the user at all. If the hearing device is not inserted in the ear, the audio processing may be turned off to save battery power.

The hearing device may comprise one or more antennas for radio frequency communication. The one or more antennas may be configured to operate in a first frequency range, such as at a frequency above 800 MHz, such as at a frequency above 1 GHz, such as at a frequency of 2.4 GHz, such as at a frequency between 1.5 GHz and 3 GHz, during use. Thus, the first antenna may be configured for operation in ISM frequency band. The first antenna may be any antenna capable of operating at these frequencies, and the first antenna may be a resonant antenna, such as monopole antenna, such as a dipole antenna, etc. The resonant antenna may have a length of lambda/4 or any multiple thereof, lambda being the wavelength corresponding to the emitted electromagnetic field.

The hearing device may comprise one or more wireless communications unit(s) or radios. The one or more wireless communications unit(s) are configured for wireless data communication, and in this respect interconnected with the one or more antennas for emission and reception of an electromagnetic field. Each of the one or more wireless communication unit may comprise a transmitter, a receiver, a transmitter-receiver pair, such as a transceiver, a radio unit, etc. The one or more wireless communication units may be configured for communication using any protocol as known for a person skilled in the art, including Bluetooth, WLAN standards, manufacture specific protocols, such as tailored proximity antenna protocols, such as proprietary protocols, such as low-power wireless communication protocols, RF communication protocols, magnetic induction protocols, etc. The one or more wireless communication units may be configured for communication using same communication protocols, or same type of communication protocols, or the one or more wireless communication units may be configured for communication using different communication protocols.

The invention also provides a system according to claim 10.

The invention also provides an application according to claim 11.

In some embodiments, the method comprises:
subsequently to providing the notification regarding adjusting the position of the first physiologic sensor:
- receiving further first physiologic sensor data from the first physiologic sensor;
- comparing the further first physiologic sensor data from the first physiologic sensor with the second physiologic sensor data from the second physiologic sensor, and when determining that the first physiologic sensor data match the second physiologic sensor data; then setting the reference physiologic sensor data based on the first physiologic and/or the second physiologic sensor data.

The method of receiving first physiologic sensor data and comparing with the second physiologic sensor data and determining whether the data match may be an iterative process which is repeated one or more times until the first physiologic sensor data and the second physiologic sensor data are determined to match. At each repetition of these methods steps, the user may be notified to adjust the position of the first physiologic sensor in/on/at his/her ear in order to obtain a better position of the first physiologic sensor such that reliable first physiologic sensor data can be obtained.

In some embodiments, the first physiologic sensor is a photoplethysmographic (PPG) sensor. A photoplethysmogram is an optically obtained plethysmogram that can be used to detect blood volume changes in the microvascular bed of tissue. A photoplethysmogram may be obtained by using a pulse oximeter which illuminates the skin and measures changes in light absorption. A conventional pulse oximeter monitors the perfusion of blood to the dermis and subcutaneous tissue of the skin. With each cardiac cycle the heart pumps blood to the periphery. Even though this pressure pulse is somewhat damped by the time it reaches the skin, it is enough to distend the arteries and arterioles in the subcutaneous tissue. If the pulse oximeter is attached without compressing the skin, a pressure pulse can also be seen from the venous plexus, as a small secondary peak. The change in volume caused by the pressure pulse may be detected by illuminating the skin with the light from a light-emitting diode (LED) and then measuring the amount of light either transmitted or reflected to a photodiode. This system is a PPG sensor. Each cardiac cycle appears as a peak. Because blood flow to the skin can be modulated by multiple other physiological systems, the PPG sensor can also be used to monitor breathing, hypovolemia, and other circulatory conditions.

Additionally, the shape of the photoplethysmographic waveform may differ from user/person to user/person, and may vary with the location and manner in which the PPG sensor is attached. The photoplethysmograms can be obtained from transmissive absorption, as at the fingertip, or reflection. In the PPG sensor, light may be transmitted to and from the skin of the user.

In some embodiments the method comprises determining a resting heart rate value of the user based on the reference physiologic sensor data. The resting heart rate value may be equal to or correspond to the reference physiologic sensor data. A resting heart rate value may be defined as being between 60 to 100 beats per minute.

In some embodiments, the method comprises detecting abnormal heart conditions based on when the first physiologic sensor in the hearing device monitors that the current heart rate of the user, wearing the hearing device, differs from the resting heart rate value. The current heart rate of the user may be monitored over a predetermined time period. Detecting whether the current heart rate of the user differs from the resting heart rate value may be by detecting whether the current heart rate differs with a predetermined threshold value from the resting heart rate value.

It is an advantage to record the resting heart rate value of the user because thereby it may be possible to detect abnormal heart conditions. Abnormal heart conditions can for example be detected if the heart rate value differs from the resting heart rate value. Abnormal heart conditions can for example be Arrhythmia, such as Atrial fibrillation. Atrial fibrillation being a common cardiac arrhythmia and a leading risk factor for stroke. In Atrial fibrillation, the heart rate may be 100 to 175 beats per minute. An irregular pulse, i.e. heart rate, is being used as an indication of Arrhythmia.

According to claim 1, the method comprises detecting first heart rate variability (HRV) data based on the first physiologic sensor data and/or detecting second heart rate variability (HRV) data based the second physiologic sensor data.

In some embodiments, determining whether the first physiologic sensor data match the second physiologic sensor data comprises comparing the first heart rate variability (HRV) data and the second heart rate variability (HRV) data. If there is too much noise or variability between the first and the second sensor data, then there is no match. It is an advantage to detect or determine the first and/or the heart rate variability (HRV) as the heart rate variability (HRV) detection is used to determine the quality of the heart rate measurement. Thus, the heart rate variability (HRV) is derived from the measured heart rate.

In some embodiments, the hearing device comprises a gyroscope sensor, and wherein the method comprises detecting the three-dimensional (3D) position of the gyroscope sensor, when the reference physiologic sensor data are set, and setting the detected 3D position as a reference 3D position of the gyroscope sensor. The head may be in a known reference position when the hearing device is inserted into the ear. It is an advantage that the user in this way may be further guided to place the first physiologic sensor correct in/on/at his/her ear, since the user hereby also has a reference 3D position to help placing the first physiologic sensor.

In some embodiments the hearing device is configured for monitoring correct placement of the first physiologic sensor to guide the user regarding how deep into the ear the hearing device should be placed. It is an advantage that the user in this way may be further guided to place the first physiologic sensor correct in/on/at his/her ear, since the user hereby also has a reference depth position to help placing the first physiologic sensor. This may be performed by using an output signal from a photo detector arranged in the ear of the user, where the signal is proportional to the reflected electromagnetic (EM) radiation. Electromagnetic radiation may be emitted and the reflected electromagnetic radiation may be measured.

In some embodiments, the external device is the electronic device, whereby the second physiologic sensor is provided in the electronic device. Thus, the external device may be the electronic device, such as a smart phone.

Alternatively, the external device is not the electronic device, in which case the external device is e.g. a chest strap, a pulse watch, or a medical heart rate monitor. A medical heart rate monitor may be used in a hospital and placed on the user's fingertip. The second physiologic sensor in an external device may be connected to the electronic device via wireless communication, e.g. BT, via wired communication, e.g. a USB cable.

In some embodiments, the electronic device is a smartphone, and wherein the second physiologic sensor comprises a camera lens and an LED light source of the smartphone, and wherein the second physiologic sensor is configured for detecting the pulse in the user's fingertip, when the user places his/her fingertip on the camera lens and LED light source. It is an advantage that the second physiologic sensor can be provided in the electronic device as the camera of the electronic device and a LED light source of the electronic device working as e.g. a PPG sensor. The method may be as follows. A software application, e.g. app, which is installed in the smartphone is used. The app is started. The user placed his/her fingertip on the rear side of the smartphone in the area of the smartphone camera module. The LED light of this module is thereby turned on and thereby illuminating the user's fingertip. The camera of the module starts recording a video of the illuminated fingertip. After recording is finished, a peripheral pulse wave can be extracted from the video signals. Based on the peripheral pulse wave, the heart rate of the user can be determined. Recording may take seconds or minutes, such as less than 5 seconds, less than 10 seconds, less than 20 seconds, less than 40 seconds, less than 1 minute, less than 2 minutes, less than 3 minutes, less than 4 minutes, or less than 5 minutes. The smart phone may be an iPhone 4S. The pulse wave signal may be derived/extracted from the green light spectrum channel of the recorded video signal. Signals may be filtered using a bandpass filter with a lower and upper cut-off frequency of 0.5 and 7 Hz, respectively. The heartbeat detection may be based on a combination of morphology and frequency analysis of the pulse wave and applied to detect all beat-to-beat intervals (BBI). From the extracted BBI time series, indices representing the variability of heart rhythm may be calculated and analysed regarding their ability to discriminate between Atrial fibrillation (AF) and (sinus rhythm) SR. For the analysis, premature beats and other disruptions may be eliminated and corresponding points on the BBI time series may be replaced, using an algorithm for adaptive variance estimation.

See e.g. the articles:
David D. McManus, Ki.H.Chon et al: "A novel application for the detection of an irregular pulse using and iPhone 4S in patients with atrial fibrillation", Journal of Heart Rhythm Society 2012.
Lian Krivoshei et al: "Smart detection of atrial fibrillation", Europace (2017), 753-757.

In some embodiments, the first PPG sensor in the hearing device is an optical sensor comprising a light emitter and a light detector, and wherein the hearing device comprises a main body comprising window means through which light emitted by the light emitter is configured to be transmitted.

In some embodiments, the first PPG sensor in the hearing device is an optical sensor comprising a light emitter and a light detector, and wherein the hearing device comprises a main body comprising a first window through which light emitted by the light emitter is configured to be transmitted to the skin of the ear and a second window through which a reflection from the skin of the ear of the light emitted by the light emitter is configured to be transmitted to the light detector.

The first physiologic sensor, e.g. heart rate sensor or PPG sensor, of the hearing device may comprise a light emitter and a light detector. The light emitter and the light detector may be arranged in two separate cavities within the PPG sensor, e.g. the light emitter and the light detector may be separated by a wall within the PPG sensor. Light emitted from the light emitter is directed to the skin of the ear wall, e.g. concha wall or skin of the ear canal. Some of the light may be absorbed by the tissue and some may be scattered back to the light transceiver. As some of this received light is affected by the pulsating blood in the blood vessels, the heart rate can be determined. The light detector may transduce the backscattered light into a signal, where only a small part of this signal represents the heart rate. Shakings due to the user's movements may cause a lot of noise to the signal. However, a motion sensor, e.g. an inertial measurement unit (IMU) in the hearing device may be used to determine the noise signal to be subtracted in order to obtain a reliable heart rate signal. The inertial measurement unit may be an accelerometer. Under all circumstances, it is important to obtain a good and well-defined light sensing.

The window means may comprise a third window through which light emitted by a further light emitter is configured to be transmitted to the skin of the ear canal. The further light emitter may be arranged in a third separated cavity within the PPG sensor. The window means may be made of a material which blocks visible light but allows near infrared light. The window means may each be arranged on different wall section of the main body pointing in different directions. The first window and/or the third window may be arranged on a wall section being parallel and/or opposite to the wall section the second window is arranged on. The first window and/or the third window may be arranged on a wall section being perpendicular and/or adjacent to the wall section the second window is arranged on. One or more of the window means may be abutting the skin of the ear wall, so that no ambient light enters the second window and disturbs the detected/ sensed signal.

### BRIEF DESCRIPTION OF THE DRAWINGS

The above and other features and advantages will become readily apparent to those skilled in the art by the following detailed description of exemplary embodiments thereof with reference to the attached drawings, in which:
Fig. 1 schematically illustrates an example of a method configured to be performed in an electronic device.
Fig. 2 schematically illustrates a block diagram of a hearing device with a first physiologic sensor.
Fig. 3a and 3b schematically illustrate examples of a system, hearing device, electronic device, external device and method configured to be performed in the electronic device.
Fig. 4a, 4b and 4c schematically illustrate an exemplary embodiment where the electronic device is a smartphone, and where the second physiologic sensor comprises a camera lens and an LED light source of the smartphone.
Fig. 5a, 5b and 5c schematically illustrate examples of notifications provided regarding adjusting the position of the first physiologic sensor in the ear of the user.
Fig. 6 schematically illustrates a hearing device comprising a first physiologic sensor, e.g. a PPG sensor.

### DETAILED DESCRIPTION

Various embodiments are described hereinafter with reference to the figures. Like reference numerals refer to like elements throughout. Like elements will, thus, not be described in detail with respect to the description of each figure. It should also be noted that the figures are only intended to facilitate the description of the embodiments. They are not intended as an exhaustive description of the claimed invention or as a limitation on the scope of the claimed invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

Throughout, the same reference numerals are used for identical or corresponding parts.

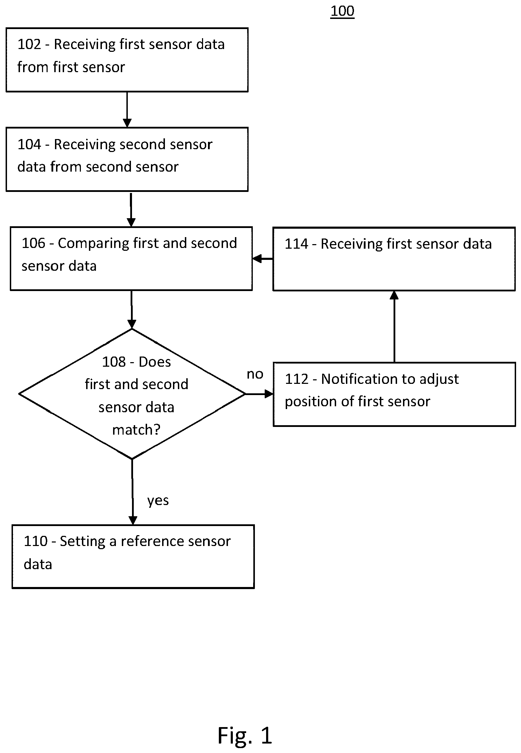

Fig. 1 schematically illustrates an example of a method 100 configured to be performed in an electronic device. The method 100 is for monitoring correct placement of a first physiologic sensor in/on/at an ear of a user. The first physiologic sensor is arranged in a hearing device, such as a hearing aid. The first physiologic sensor in the hearing device is configured for monitoring the heart rate of the user wearing the hearing device. The electronic device and the hearing device are configured to be connected. The method 100 comprises, at the electronic device, receiving 102 first physiologic sensor data from the first physiologic sensor. The method comprises, at the electronic device, receiving 104 second physiologic sensor data from a second physiologic sensor provided in an external device, where the external device is configured for detecting the heart rate of the user using the second physiologic sensor. The method comprises, at the electronic device, comparing 106 the first physiologic sensor data from the first physiologic sensor with the second physiologic sensor data from the second physiologic sensor. The method comprises, at the electronic device, determining 108 whether the first physiologic sensor data match the second physiologic sensor data. The method comprises, at the electronic device, in accordance with a determination that the first physiologic sensor data match the second physiologic sensor data, setting 110 a reference physiologic sensor data based on the first physiologic sensor data and/or the second physiologic sensor data. The method comprises, at the electronic device, in accordance with a determination that the first physiologic sensor data do not match the second physiologic sensor data, providing 112 a notification regarding adjusting the position of the first physiologic sensor in/on/at the ear of the user.

When the first physiologic sensor data do not match the second physiologic sensor data, then subsequently to providing 112 the notification regarding adjusting the position of the first physiologic sensor, the method comprises receiving 114 further first physiologic sensor data from the first physiologic sensor. The method comprises comparing 106 the further first physiologic sensor data from the first physiologic sensor with the second physiologic sensor data from the second physiologic sensor. The method comprises determining 108 whether the further first physiologic sensor data match the second physiologic sensor data. These steps 106, 108, 112 and 114 may be repeated one or more times until the first physiologic sensor data match the second physiologic sensor data. When determining that the first physiologic sensor data match the second physiologic sensor data; then the method comprises setting the reference physiologic sensor data based on the first physiologic and/or the second physiologic sensor data.

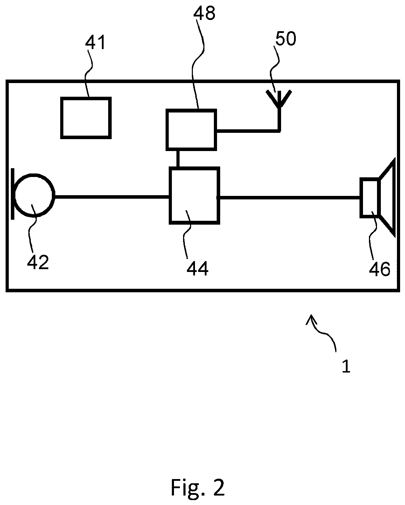

Fig. 2 schematically illustrates a block diagram of a hearing device 1, such as a hearing aid, with a first physiologic sensor 41. The first physiologic sensor 41 may be configured for being placed in/on/at the ear of a user. The first physiologic sensor 41 may be configured for being placed in the ear canal of the user. The first physiologic sensor 41 may be a PPG sensor. The hearing device 1 comprises a microphone 42, for receiving an input signal and converting it into an audio signal. The audio signal is provided to a processing unit 44 for processing the audio signal and providing a processed output signal for compensating a hearing loss of a user of the hearing device 1. A receiver 46 is connected to an output of the processing unit 44 for converting the processed output signal into an output sound signal, e.g. a signal modified to compensate for a user's hearing impairment. Typically, the receiver 46 comprises a transducer, and the receiver 46 is often referred to as a speaker. The processing unit 44 may comprise elements such as amplifiers, compressors, noise reduction systems, etc. The hearing device 1 comprises the first physiologic sensor 41. The hearing device 1 may further comprise a wireless communication unit 48 for wireless data communication interconnected with an antenna structure 50 for emission and reception of an electromagnetic field. The wireless communication unit 48, such as a radio or a transceiver, connects to the processing unit 44 and the antenna structure 50, for communicating with an electronic device, an external device, or with another hearing device, such as another hearing aid located in/on/at another ear of the user, typically in a binaural hearing system. The hearing device 1 may comprise two or more antenna structures. A first antenna may be for connecting to an electronic device, such as a smart phone of the user. A second antenna may be for connection with another hearing device, such as a hearing aid. A third antenna may be for connecting with an external device comprising a second physiologic sensor, e.g. a chest strap, a pulse watch or a medical heart rate monitor such as attached to the finger of the user, for detecting the heart rate of the user.

The hearing device 1 may be a behind-the-ear hearing device, and may be provided as a behind-the-ear module. The hearing device 1 may be an in-the-ear hearing device and may be provided as an in-the-ear module. Alternatively, parts of the hearing device 1 may be provided in a behind-the-ear module, while other parts, such as the receiver 46, may be provided in an in-the-ear module.

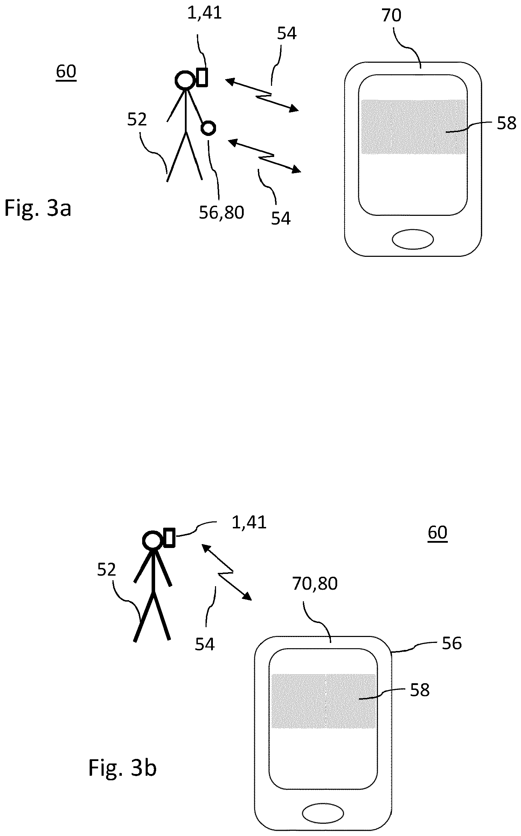

Fig. 3a schematically illustrates an example of a system 60, hearing device 1, electronic device 70, external device 80 and method configured to be performed in the electronic device 70. The method is for monitoring correct placement of a first physiologic sensor 41 in/on/at an ear of a user 52. The first physiologic sensor 41 is arranged in a hearing device 1. The first physiologic sensor 41 in the hearing device 1 is configured for monitoring the heart rate of the user 52 wearing the hearing device 1. The electronic device 70 and the hearing device 1 are configured to be connected 54, e.g. wirelessly by means of Bluetooth (BT). The method comprises, at the electronic device 70, receiving first physiologic sensor data from the first physiologic sensor 41. The method comprises, at the electronic device 70, receiving second physiologic sensor data from a second physiologic sensor 56 provided in the external device 80, where the external device 80 is configured for detecting the heart rate of the user 52 using the second physiologic sensor 56. The electronic device 70 and the external device 80 are configured to be connected 54, e.g. wirelessly by means of Bluetooth (BT). The method comprises, at the electronic device 70, comparing the first physiologic sensor data from the first physiologic sensor 41 with the second physiologic sensor data from the second physiologic sensor 56. The method comprises, at the electronic device 70, determining whether the first physiologic sensor data match the second physiologic sensor data. The method comprises, at the electronic device 70, in accordance with a determination that the first physiologic sensor data match the second physiologic sensor data, setting a reference physiologic sensor data based on the first physiologic sensor data and/or the second physiologic sensor data. The method comprises, at the electronic device 70, in accordance with a determination that the first physiologic sensor data do not match the second physiologic sensor data, providing a notification 58 regarding adjusting the position of the first physiologic sensor 41 in/on/at the ear of the user 52.

A software application, e.g. app, which is installed in the electronic device 70 may be used. The app is started. The app starts listening for and/or requesting for the first physiologic sensor data form the hearing device 1 and the second physiologic sensor data from the external device 80. When the software application receives the first physiologic sensor data and the second physiologic sensor data, the software application may initiate the method described above.

The second physiologic sensor data may be obtained from a different physical point or part or location on the user's 52 body or skin than where the first physiologic sensor data is obtained. The first physiologic sensor data is obtained in/on/at the ear of the user 52. The second physiologic sensor data may be obtained e.g. at the chest or wrist or finger of the user 52. The first physiologic sensor data and the second physiologic sensor data are configured to be detected simultaneously.

Fig. 3a illustrates an exemplary embodiment where the external device 80 can be a pulse watch or medical finger sensor which is configured for obtaining physiologic sensor data of the user. The pulse watch is configured to obtain physiologic sensor data from the wrist or the skin of the wrist of the user.

The external device 80 may be a medical finger sensor which is configured for obtaining physiologic sensor data of the user 52. The medical finger sensor is configured to obtain physiologic sensor data from the finger or the skin of the finger of the user. A medical finger sensor is typically used to measure a patient's heart rate by medical doctors in hospitals.

The external device 80 may alternatively be a chest strap which is configured to obtain physiologic sensor data from the chest or the skin of the chest of the user. Chest straps and pulse watches are typically used by a user to measure his/her own heart rate when performing sports.

Fig. 3b shows an exemplary embodiment where the external device 80 may be the electronic device 70, e.g. a smartphone, tablet, pc, computer etc., in which case the electronic device 70 is configured for obtaining the second physiologic sensor data of the user 52.

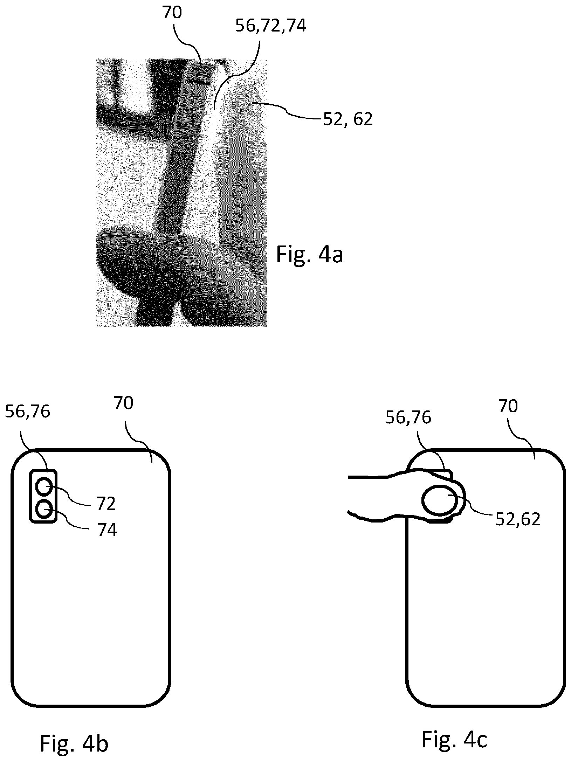

When the external device 80 is the electronic device 70, the second physiologic sensor 56 is provided in the electronic device 70. The electronic device 70 may be a smartphone, and the second physiologic sensor 56 may comprise a camera lens and an LED light source, see fig. 4a, 4b and 4c, of the electronic device 70, e.g. smartphone. The second physiologic sensor 56 may be configured for detecting the pulse in the user's fingertip, when the user 52 places his/her fingertip on the camera lens and LED light source, see fig. 4a, 4b and 4c.

Fig. 4a, 4b and 4c schematically illustrate an exemplary embodiment where the electronic device 70 is a smartphone, and where the second physiologic sensor 56 comprises a camera lens 72 and an LED light source 74 of the smartphone 70. The second physiologic sensor 56 is configured for detecting the pulse in the user's 52 fingertip 62, when the user 52 places his/her fingertip 62 on the camera lens 72 and LED light source 74. The second physiologic sensor 56 can thus be provided in the electronic device 70 as the camera 72 of the electronic device 70 and a LED light source 74 of the electronic device 70 are working as e.g. a PPG sensor. The method for detecting the second physiologic sensor data may be as follows. A software application, e.g. app, which is installed in the smartphone 70 is used. The app is started. The app starts listening for and/or requesting for the first physiologic sensor data form the hearing device 1 and the second physiologic sensor data from the electronic device 70. The user 52 places his/her fingertip 62 in the area of the smartphone's 70 camera module 76. The camera module 76 comprising the LED light source 74 and the camera lens 72. The camera module 76 may be placed on the rear side of the smartphone 70. The LED light 74 of the camera module 76 is thereby turned on and thereby illuminating the user's fingertip 62. Through the camera lens 72 the camera module 76 starts recording a video of the illuminated fingertip 62. After recording is finished, a peripheral pulse wave can be extracted from the video signals. Based on the peripheral pulse wave, the heart rate of the user 52 can be determined. Recording may take seconds or minutes, such as less than 5 seconds, less than 10 seconds, less than 20 seconds, less than 40 seconds, less than 1 minute, less than 2 minutes, less than 3 minutes, less than 4 minutes, or less than 5 minutes. The smart phone 70 may be an iPhone 4S. The pulse wave signal may be derived/extracted from a green light spectrum channel of the recorded video signal. Signals may be filtered using a bandpass filter with a lower and upper cut-off frequency of 0.5 and 7 Hz, respectively. The heartbeat detection may be based on a combination of morphology and frequency analysis of the pulse wave and applied to detect all beat-to-beat intervals (BBI). From the extracted BBI time series, indices representing the variability of heart rhythm may be calculated and analysed regarding their ability to discriminate between Atrial fibrillation (AF) and (sinus rhythm) SR. For the analysis, premature beats and other disruptions may be eliminated and corresponding points on the BBI time series may be replaced, using an algorithm for adaptive variance estimation. When the software application receives the first physiologic sensor data and the second physiologic sensor data, the software application may initiate the method 100 described with Fig. 1.

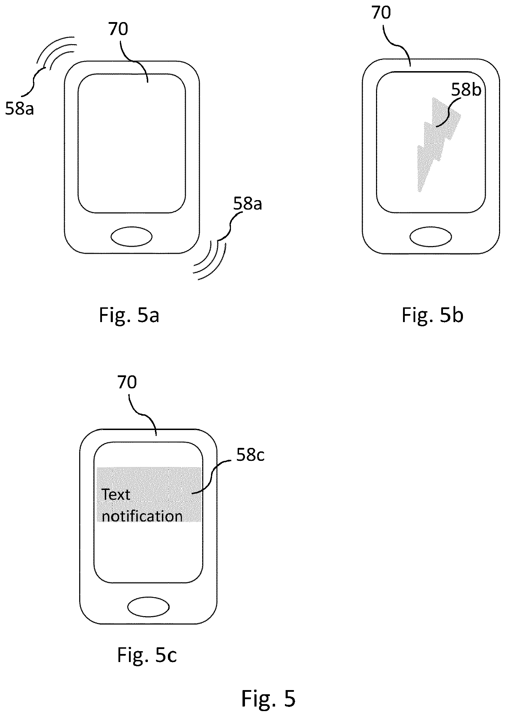

Fig. 5a, 5b and 5c schematically illustrates examples of notifications 58, also referred to as 58a, 58b and 58c, provided regarding adjusting the position of the first physiologic sensor in/on/at the ear of the user. The method in the electronic device 70 comprises in accordance with a determination that the first physiologic sensor data do not match the second physiologic sensor data, providing a notification 58 regarding adjusting the position of the first physiologic sensor in/on/at the ear of the user. If the first physiologic sensor data and the second physiologic sensor data do not match, it may be assumed that the first physiologic sensor is not correctly placed in/on/at the ear of the user. A notification 58 in the electronic device 70 may be provided to the user. The notification 58 may comprise instructions to the user for adjusting the position of the first physiologic sensor in/on/at his/her ear. The first physiologic sensor may be implemented in the hearing device, thus the notification to the user may regard adjusting the position of the hearing device, whereby the first physiologic sensor in the hearing device is also adjusted in position relative to the ear of the user. The purpose of the notification 58 is to make the user adjust the position of the first physiologic sensor, such that the first physiologic sensor is placed correctly to monitor the heart rate in the ear of the user.

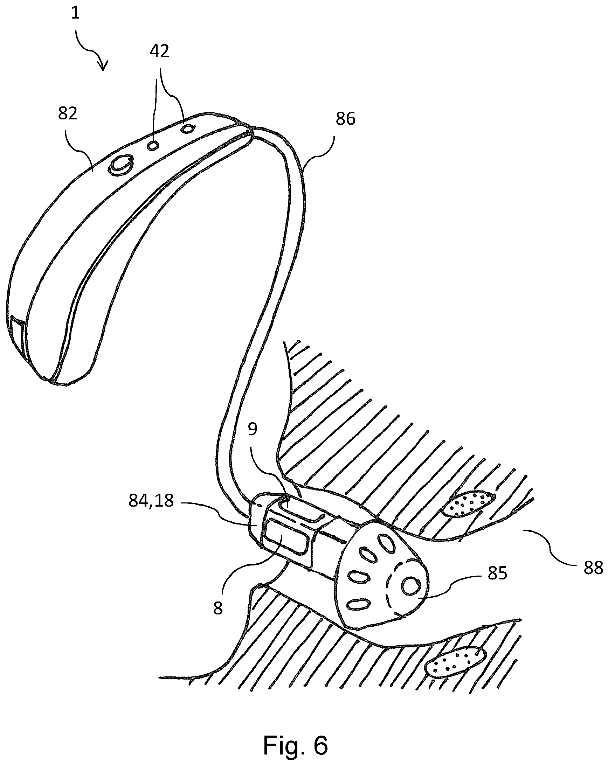

The notification 58 may be in the form of a recommendation, an error message, and/or instructions to adjust the position of the first physiologic sensor in the ear and/or adjust the position of the hearing device in the ear. The notification 58 may be instructions to the user to change a fitting member, see fig. 6, of the hearing device to obtain a better fit of the hearing device in the ear. The notification 58 may be directed to the user or to a hearing care professional (HCP) who is assisting the user with the hearing device. The notification 58 may be instructions 58c in a first user interface of the electronic device 70, e.g. a graphical user interface on the user's smartphone 70, which is connected to the hearing device. The notification 58 may be visual, audio and/or tactile. The notification 58 may comprise visual, audio and/or tactile notifications 58.

Fig. 5a shows an example of a tactile notification 58a which may be a haptic feedback or a vibration alerting the user that the hearing device, and thereby the first physiologic sensor, is not correctly placed in the ear. The tactile notification 58a may be generated by a tactile output generator in the electronic device 70.

Fig. 5b) shows an example of an audio notification 58b which may be a sound alerting the user that the hearing device, and thereby the first physiologic sensor, is not correctly placed in the ear. The sound may be a tone or a voice speaking. The audio notification 58b may be transmitted from a loudspeaker in the electronic device 70.

Fig. 5c) shows an example of a visual notification 58c which may be a text alert stating e.g. "Please adjust position of your hearing device in the ear for improved monitoring of heart rate". A visual notification 58c may be a text message stating e.g. "Please push your hearing device further into your ear canal", and/or "Please move your hearing device closer to an ear wall/the concha wall/concha skin". A visual notification 58c may comprise a picture of the desired orientation of the hearing device, e.g. showing that the end/side/face of the hearing device, where the first physiologic sensor is arranged, should point towards a specific ear wall, e.g. the concha wall or concha skin, in the ear. The visual notification 58c may be displayed in a graphical user interface of the electronic device 70.

For obtaining reliable first physiologic sensor data and second physiologic sensor data, the user should sit or lie still, i.e. the user should not be moving, when the first physiologic sensor data and the second physiologic sensor data are obtained or measured. Thus, if the first physiologic sensor data does not match the second physiologic sensor data it may be because the user is not sitting still but is moving. Thus, the notification 58 may comprise instructions to the user to sit or lie still.

Fig. 6 schematically illustrates a hearing device 1 comprising a first physiologic sensor 41 (not shown), e.g. a PPG sensor. The hearing device 1 comprises a behind-the-ear (BTE) unit 82 and an in the ear unit 84. In the BTE unit 82, the microphone(s) 42 and the processing unit 44 (not shown) may be arranged. The BTE unit 82 is configured to be arranged behind the ear of the user. A wire or cable or tube 86 connects the BTE unit 82 with the in the ear unit 84. The in the ear unit 84 may comprise a receiver 46 (not shown). The in the ear unit 84 may comprise a fitting member 85, such as an earpiece or dome. The fitting member 85 may be covering and/or connected to the receiver 46 (not shown). The in the ear unit 84 comprises the first physiologic sensor 41. The first physiologic sensor 41 is configured to be arranged in the ear canal 88 for measuring the first physiologic sensor data. The first physiologic sensor 41 may be a PPG sensor measuring the heart rate of the user. A PPG sensor is an optical sensor comprising a light emitter (LED) and a light detector. The first physiologic sensor 41 comprises a main body 18. The main body 18 comprises window means, such as a first window 8 through which light emitted by a light emitter (LED) is configured to be transmitted and a second window 9 through which a reflection from the light emitted by the light emitter (LED) is configured to be detected by a light detector. The window means 8, 9 may be made of a material which blocks visible light but allows near infrared light. Thus, the main body 18 also comprises the light detector for detecting the reflected light from the skin. Furthermore, the main body 18 may comprise an inertial measurement unit (IMU), such as an accelerometer.

Although particular features have been shown and described, it will be understood that they are not intended to limit the claimed invention, and it will be made obvious to those skilled in the art that various changes and modifications may be made without departing from the scope of the claimed invention.

### LIST OF REFERENCES

1 hearing device
8 first window
9 second window
18 main body
41 first physiologic sensor
42 microphone
44 processing unit
46 receiver
48 wireless communication unit
50 antenna structure
52 user
54 wireless connection
56 second physiologic sensor
58 notification
60 system
70 electronic device
72 camera lens
74 LED light source
76 camera module
80 external device
82 behind-the-ear (BTE) unit
84 in ear unit
85 fitting member
86 cable, wire, tube
88 ear canal
100 method
102 receiving first physiologic sensor data from the first physiologic sensor
104 receiving second physiologic sensor data from a second physiologic sensor
106 comparing the first physiologic sensor data from the first physiologic sensor with the second physiologic sensor data from the second physiologic sensor
108 determining whether the first physiologic sensor data match the second physiologic sensor data
110 setting a reference physiologic sensor data based on the first physiologic sensor data and/or the second physiologic sensor data
112 providing a notification regarding adjusting the position of the first physiologic sensor in the ear of the user
114 receiving further first physiologic sensor data from the first physiologic sensor

## Claims

1. A method in an electronic device, the method is for monitoring correct placement of a first physiologic sensor in/on/at an ear of a user, the first physiologic sensor is arranged in a hearing device, the first physiologic sensor in the hearing device is configured for monitoring the heart rate of the user wearing the hearing device, the electronic device and the hearing device being connected, the method comprises at the electronic device:
- receiving first physiologic sensor data from the first physiologic sensor;
- receiving second physiologic sensor data from a second physiologic sensor provided in an external device, where the external device is configured for detecting the heart rate of the user using the second physiologic sensor;
- comparing the first physiologic sensor data from the first physiologic sensor with the second physiologic sensor data from the second physiologic sensor;
- determining whether the first physiologic sensor data match the second physiologic sensor data;
- in accordance with a determination that the first physiologic sensor data match the second physiologic sensor data, setting a reference physiologic sensor data based on the first physiologic sensor data and/or the second physiologic sensor data;
- in accordance with a determination that the first physiologic sensor data do not match the second physiologic sensor data, providing a notification regarding adjusting the position of the first physiologic sensor in/on/at the ear of the user; and
wherein the method further comprises detecting first heart rate variability, HRV, data based on the first physiologic sensor data and/or detecting second heart rate variability, HRV, data based the second physiologic sensor data.

2. The method according to claim 1, wherein the method comprises:
subsequently to providing the notification regarding adjusting the position of the first physiologic sensor:
- receiving further first physiologic sensor data from the first physiologic sensor;
- comparing the further first physiologic sensor data from the first physiologic sensor with the second physiologic sensor data from the second physiologic sensor, and when determining that the first physiologic sensor data match the second physiologic sensor data; then setting the reference physiologic sensor data based on first physiologic sensor data and/or the second physiologic sensor data.

3. The method according to any of the preceding claims, wherein the first physiologic sensor is a photoplethysmographic, PPG, sensor.

4. The method according to any of the preceding claims, wherein the method comprises determining a resting heart rate value of the user based on the reference physiologic sensor data.

5. The method according to any of the preceding claims, wherein determining whether the first physiologic sensor data match the second physiologic sensor data comprises comparing the first heart rate variability, HRV, data and the second heart rate variability, HRV, data.

6. The method according to any of the preceding claims, where the hearing device comprises a gyroscope sensor, and wherein the method comprises detecting the three-dimensional, 3D, position of the gyroscope sensor, when the reference physiologic sensor data are set, and setting the detected 3D position as a reference 3D position of the gyroscope sensor.

7. The method according to any of the preceding claims, wherein the external device is the electronic device, whereby the second physiologic sensor is provided in the electronic device.

8. The method according to the preceding claim, wherein the electronic device is a smartphone, and wherein the second physiologic sensor comprises a camera lens and an LED light source of the smartphone, and wherein the second physiologic sensor is configured for detecting the pulse in the user's fingertip, when the user places his/her fingertip on the camera lens and LED light source.

9. The method according to claim 3, wherein the first PPG sensor in the hearing device is an optical sensor comprising a light emitter and a light detector, and wherein the hearing device comprises a main body comprising a first window through which light emitted by the light emitter is configured to be transmitted to the skin of the ear, and a second window through which a reflection from the skin of the ear of the light emitted by the light emitter is configured to be transmitted to the light detector.

10. A system comprising a hearing device, the hearing device comprising a first physiological sensor, the first physiologic sensor being configured for monitoring the hear rate in/on/at the ear of a user wearing the hearing device, wherein the hearing device is configured to be connected with an electronic device, and the hearing device is configured to transmit at least a first physiologic sensor data from the first physiologic sensor to the electronic device, for monitoring the correct placement of the first physiologic sensor in/ on/at the ear of the user; and the electronic device is configured with an application installed/run thereon, the application when run by the electronic device is configured to perform on the electronic device the steps of claim 1.

11. An application configured to be installed/run in an electronic device, the application is configured, when run by the electronic device, to perform on the electronic device the steps of claim 1.

## Patentansprüche

1. Verfahren in einer elektronischen Vorrichtung, wobei das Verfahren zum Überwachen der korrekten Platzierung eines ersten physiologischen Sensors in/auf/an einem Ohr eines Benutzers dient, wobei der erste physiologische Sensor in einer Hörvorrichtung angeordnet ist, wobei der erste physiologische Sensor in der Hörvorrichtung zum Überwachen der Herzfrequenz des Benutzers, der die Hörvorrichtung trägt, konfiguriert ist, wobei die elektronische Vorrichtung und die Hörvorrichtung verbunden sind, wobei das Verfahren Folgendes an der elektronischen Vorrichtung umfasst:
- Empfangen von ersten physiologischen Sensordaten von dem ersten physiologischen Sensor;
- Empfangen von zweiten physiologischen Sensordaten von einem zweiten physiologischen Sensor, der in einer externen Vorrichtung bereitgestellt ist, wobei die externe Vorrichtung zum Detektieren der Herzfrequenz des Benutzers unter Verwendung des zweiten physiologischen Sensors konfiguriert ist;
- Vergleichen der ersten physiologischen Sensordaten von dem ersten physiologischen Sensor mit den zweiten physiologischen Sensordaten von dem zweiten physiologischen Sensor;
- Bestimmen, ob die ersten physiologischen Sensordaten mit den zweiten physiologischen Sensordaten übereinstimmen;
- gemäß einer Bestimmung, dass die ersten physiologischen Sensordaten mit den zweiten physiologischen Sensordaten übereinstimmen, Festlegen von physiologischen Referenzsensordaten basierend auf den ersten physiologischen Sensordaten und/oder den zweiten physiologischen Sensordaten;
- gemäß einer Bestimmung, dass die ersten physiologischen Sensordaten nicht mit den zweiten physiologischen Sensordaten übereinstimmen, Bereitstellen einer Benachrichtigung hinsichtlich eines Einstellens der Position des ersten physiologischen Sensors in/auf/an dem Ohr des Benutzers; und
wobei das Verfahren ferner Detektieren von ersten Herzfrequenzvariabilitätsdaten, HRV-Daten, basierend auf den ersten physiologischen Sensordaten und/oder Detektieren von zweiten Herzfrequenzvariabilitätsdaten, HRV-Daten, basierend auf den zweiten physiologischen Sensordaten umfasst.

2. Verfahren nach Anspruch 1, wobei das Verfahren Folgendes umfasst:
im Anschluss an das Bereitstellen der Benachrichtigung hinsichtlich des Einstellens der Position des ersten physiologischen Sensors:
- Empfangen von weiteren ersten physiologischen Sensordaten von dem ersten physiologischen Sensor;
- Vergleichen der weiteren ersten physiologischen Sensordaten von dem ersten physiologischen Sensor mit den zweiten physiologischen Sensordaten von dem zweiten physiologischen Sensor; und
wenn bestimmt wird, dass die ersten physiologischen Sensordaten mit den zweiten physiologischen Sensordaten übereinstimmen; dann Festlegen der physiologischen Referenzsensordaten basierend auf den ersten physiologischen Sensordaten und/oder den zweiten physiologischen Sensordaten.

3. Verfahren nach einem der vorhergehenden Ansprüche, wobei der erste physiologische Sensor ein photoplethysmographischer Sensor, PPG-Sensor, ist.

4. Verfahren nach einem der vorhergehenden Ansprüche, wobei das Verfahren Bestimmen eines Ruheherzfrequenzwerts des Benutzers basierend auf den physiologischen Referenzsensordaten umfasst.

5. Verfahren nach einem der vorhergehenden Ansprüche, wobei das Bestimmen, ob die ersten physiologischen Sensordaten mit den zweiten physiologischen Sensordaten übereinstimmen, Vergleichen der ersten Herzfrequenzvariabilitätsdaten, HRV-Daten, und der zweiten Herzfrequenzvariabilitätsdaten, HRV-Daten, umfasst.

6. Verfahren nach einem der vorhergehenden Ansprüche, wobei die Hörvorrichtung einen Gyroskopsensor umfasst und wobei das Verfahren Detektieren der dreidimensionalen Position, 3D-Position, des Gyroskopsensors, wenn die physiologischen Referenzsensordaten festgelegt sind, und Festlegen der detektierten 3D-Position als 3D-Referenzposition des Gyroskopsensors umfasst.

7. Verfahren nach einem der vorhergehenden Ansprüche, wobei die externe Vorrichtung die elektronische Vorrichtung ist, wodurch der zweite physiologische Sensor in der elektronischen Vorrichtung bereitgestellt ist.

8. Verfahren nach dem vorhergehenden Anspruch, wobei die elektronische Vorrichtung ein Smartphone ist und wobei der zweite physiologische Sensor ein Kameraobjektiv und eine LED-Lichtquelle des Smartphones umfasst und wobei der zweite physiologische Sensor zum Detektieren des Pulses in der Fingerspitze des Benutzers konfiguriert ist, wenn der Benutzer seine Fingerspitze auf das Kameraobjektiv und die LED-Lichtquelle legt.

9. Verfahren nach Anspruch 3, wobei der erste PPG-Sensor in der Hörvorrichtung ein optischer Sensor ist, der einen Lichtsender und einen Lichtdetektor umfasst, und wobei die Hörvorrichtung einen Hauptkörper umfasst, umfassend ein erstes Fenster, durch das durch den Lichtsender emittiertes Licht dazu konfiguriert ist, auf die Haut des Ohrs übertragen zu werden, und ein zweites Fenster, durch das eine Reflexion von der Haut des Ohrs des durch den Lichtsender emittierten Lichts dazu konfiguriert ist, auf den Lichtdetektor übertragen zu werden.

10. System, umfassend eine Hörvorrichtung, wobei die Hörvorrichtung einen ersten physiologischen Sensor umfasst, wobei der erste physiologische Sensor zum Überwachen der Herzfrequenz in/auf/an dem Ohr eines Benutzers, der die Hörvorrichtung trägt, konfiguriert ist, wobei die Hörvorrichtung dazu konfiguriert ist, mit einer elektronischen Vorrichtung verbunden sein, und die Hörvorrichtung dazu konfiguriert ist, mindestens erste physiologische Sensordaten zum Überwachen der korrekten Platzierung des ersten physiologischen Sensors in/auf/an dem Ohr des Benutzers von dem ersten physiologischen Sensor an die elektronische Vorrichtung zu übertragen; und die elektronische Vorrichtung mit einer darauf installierten/ausgeführten Anwendung konfiguriert ist, wobei die Anwendung, wenn sie durch die elektronische Vorrichtung ausgeführt wird, dazu konfiguriert ist, auf der elektronischen Vorrichtung die Schritte nach Anspruch 1 durchzuführen.

11. Anwendung, die dazu konfiguriert ist, in einer elektronischen Vorrichtung installiert/ausgeführt zu werden, wobei die Anwendung dazu konfiguriert ist, wenn sie durch die elektronische Vorrichtung ausgeführt wird, auf der elektronischen Vorrichtung die Schritte nach Anspruch 1 durchzuführen.

## Revendications

1. Procédé dans un dispositif électronique, le procédé consiste à surveiller le placement correct d'un premier capteur physiologique dans/sur/au niveau d'une oreille d'un utilisateur, le premier capteur physiologique est agencé dans un appareil auditif, le premier capteur physiologique dans l'appareil auditif est configuré pour surveiller la fréquence cardiaque de l'utilisateur portant l'appareil auditif, le dispositif électronique et l'appareil auditif étant connectés, le procédé comprend au niveau du dispositif électronique :
- la réception de premières données de capteur physiologique provenant du premier capteur physiologique ;
- la réception de secondes données de capteur physiologique provenant d'un second capteur physiologique prévu dans un dispositif externe, dans lequel le dispositif externe est configuré pour détecter la fréquence cardiaque de l'utilisateur à l'aide du second capteur physiologique ;
- la comparaison des premières données de capteur physiologique provenant du premier capteur physiologique avec les secondes données de capteur physiologique provenant du second capteur physiologique ;
- le fait de déterminer si les premières données de capteur physiologique correspondent aux secondes données de capteur physiologique ;
- conformément à une détermination selon laquelle les premières données de capteur physiologique correspondent aux secondes données de capteur physiologique, le fait de définir des données de capteur physiologique de référence sur la base des premières données de capteur physiologique et/ou des secondes données de capteur physiologique ;
- conformément à une détermination selon laquelle les premières données de capteur physiologique ne correspondent pas aux secondes données de capteur physiologique, la fourniture d'une notification concernant l'ajustement de la position du premier capteur physiologique dans/sur/au niveau de l'oreille de l'utilisateur ; et
dans lequel le procédé comprend en outre la détection de premières données de variabilité de fréquence cardiaque, HRV, sur la base des premières données de capteur physiologique et/ou la détection de secondes données de variabilité de fréquence cardiaque, HRV, sur la base des secondes données de capteur physiologique.

2. Procédé selon la revendication 1, dans lequel le procédé comprend : après la fourniture de la notification concernant l'ajustement de la position du premier capteur physiologique :
- la réception d'autres premières données de capteur physiologique provenant du premier capteur physiologique ;
- la comparaison des autres premières données de capteur physiologique provenant du premier capteur physiologique avec les secondes données de capteur physiologique provenant du second capteur physiologique, et lors de la détermination que les premières données de capteur physiologique correspondent aux secondes données de capteur physiologique ; le fait de définir ensuite les données de capteur physiologique de référence sur la base des premières données de capteur physiologique et/ou des secondes données de capteur physiologique.

3. Procédé selon l'une quelconque des revendications précédentes, dans lequel le premier capteur physiologique est un capteur photopléthysmographique, PPG.

4. Procédé selon l'une quelconque des revendications précédentes, dans lequel le procédé comprend la détermination d'une valeur de fréquence cardiaque au repos de l'utilisateur sur la base des données de capteur physiologique de référence.

5. Procédé selon l'une quelconque des revendications précédentes, dans lequel le fait de déterminer si les premières données de capteur physiologique correspondent aux secondes données de capteur physiologique comprend la comparaison des premières données de variabilité de fréquence cardiaque, HRV, et des secondes données de variabilité de fréquence cardiaque.

6. Procédé selon l'une quelconque des revendications précédentes, dans lequel l'appareil auditif comprend un capteur de gyroscope, et dans lequel le procédé comprend la détection de la position tridimensionnelle, 3D, du capteur de gyroscope, lorsque les données de capteur physiologique de référence sont définies, et le fait de définir la position 3D détectée comme position 3D de référence du capteur de gyroscope.

7. Procédé selon l'une quelconque des revendications précédentes, dans lequel le dispositif externe est le dispositif électronique, moyennant quoi le second capteur physiologique est prévu dans le dispositif électronique.

8. Procédé selon la revendication précédente, dans lequel le dispositif électronique est un smartphone, et dans lequel le second capteur physiologique comprend un objectif de caméra et une source de lumière DEL du smartphone, et dans lequel le second capteur physiologique est configuré pour détecter le pouls dans le bout du doigt de l'utilisateur, lorsque l'utilisateur place le bout de son doigt sur l'objectif de la caméra et la source de lumière DEL.

9. Procédé selon la revendication 3, dans lequel le premier capteur PPG dans l'appareil auditif est un capteur optique comprenant un émetteur de lumière et un détecteur de lumière, et dans lequel l'appareil auditif comprend un corps principal comprenant une première fenêtre à travers laquelle la lumière émise par l'émetteur de lumière est configurée pour être transmise à la peau de l'oreille, et une seconde fenêtre à travers laquelle une réflexion depuis la peau de l'oreille de la lumière émise par l'émetteur de lumière est configurée pour être transmise au détecteur de lumière.

10. Système comprenant un appareil auditif, l'appareil auditif comprenant un premier capteur physiologique, le premier capteur physiologique étant configuré pour surveiller la fréquence cardiaque dans/sur/au niveau de l'oreille d'un utilisateur portant l'appareil auditif, dans lequel l'appareil auditif est configuré pour être connecté à un dispositif électronique, et l'appareil auditif est configuré pour transmettre au moins des premières données de capteur physiologique provenant du premier capteur physiologique au dispositif électronique, pour surveiller le placement correct du premier capteur physiologique dans/sur/au niveau de l'oreille de l'utilisateur ; et le dispositif électronique est configuré avec une application installée/exécutée dessus, l'application, lorsqu'elle est exécutée par le dispositif électronique, est configurée pour exécuter, sur le dispositif électronique, les étapes selon la revendication 1.

11. Application configurée pour être installée/exécutée sur un dispositif électronique, l'application est configurée, lorsqu'elle est exécutée sur le dispositif électronique, pour exécuter sur le dispositif électronique les étapes selon la revendication 1.
